# EUROPEAN PATENT SPECIFICATION

(11) **EP 3 829 472 B1**
(45) Date of publication and mention of the grant of the patent: **17.01.2024**
(21) Application number: 19752373.1
(22) Date of filing: 30.07.2019
(51) Int. Cl.: A61B 18/04, A61N 1/44, H05H 1/24, A61N 1/04, A61N 1/32, A61N 5/02, A61N 5/06

(54) **COLD PLASMA GENERATING DEVICES AND SYSTEMS**
KALTPLASMAERZEUGUNGSVORRICHTUNGEN UND -SYSTEME
DISPOSITIFS ET SYSTÈMES DE GÉNÉRATION DE PLASMA FROID

(30) Priority: 31.07.2018 US 201862712812 P; 31.07.2018 US 201862712849 P; 31.07.2018 US 201862712860 P; 31.07.2018 US 201862712873 P; 31.07.2018 US 201862712876 P
(43) Date of publication of application: 09.06.2021
(73) Proprietor: L'OREAL, 75008 Paris (FR); Deane, Geoffrey, Redmond WA 98052 (US)
(72) Inventor: DEANE, Geoffrey, Redmond, Washington 98052 (US); YILDIRIM, Ozgur Emek, Redmond, Washington 98052 (US); PLANARD-LUONG, Thi Hong Lien, Redmond, Washington 98052 (US); JACOB, Matthieu, Redmond, Washington 98052 (US)
(74) Representative: Casalonga
(86) International application number: PCT/US2019/044083
(87) International publication number: WO 2020/028329

(56) References cited:
- EP-A1- 3 068 328
- US-A1- 2014 188 097
- US-A1- 2018 008 333

## Description

The present invention relates to a cold plasma system for cosmetic treatment according to the preamble of claim 1 such as it is e.g. know from US 2018/008333 A1, EP 3 068 328 A1 and US 2014/188097 A1.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

Portable cosmetic devices incorporating cold atmospheric plasma (additionally referred to as "cold plasma" or "plasma"), as currently available, implement limited modalities of treatment. Most focus solely on generating a plasma in proximity to a region of a person's skin as an avenue for treatment of the region. Such an approach does not leverage the therapeutic advantages and benefits of a multi-modal treatment and does not create synergistic therapy outcomes that exceed the effect of the individual treatment modalities in isolation. In some embodiments of the present technology, a multi-modal cold plasma device includes one or more auxiliary or additional mechanisms (vibration, heat, etc.) in addition to generating the cold plasma.

A multi-modal plasma device that takes advantage of synergistic treatment effects will achieve enhanced results over a shortened treatment duration and a reduced power demand, relative to a mono-modal plasma device. What is more, a multi-modal device mitigates the potential risks of excessive exposure to potentially harmful species generated in the plasma, such as oxidizing species or reactive radicals, by enhancing permeability of plasma generated species, or by providing secondary treatment that does not rely on plasma generated species for efficacy.

Blemishes develop for multiple reasons, some of which include harmful bacteria, blocked pores, irritation, imbalance in moisture or oil, etc. Typically, blemishes develop from an easily treatable early stage to a painful and difficult condition within a single day, often within hours of first sensing a new blemish. Without being bound to theory, it is believed that exposure to a cold atmospheric plasma and species generated therein will effectively treat blemishes. It is further believed that rapid treatment at the site of the blemish, immediately upon detection, prevents the growth of unsightly blemishes that may leave permanent scarring.

Cold plasma devices designed to treat large regions of the skin, incorporating internal power sources sufficient for prolonged use, are too large and too heavy to conveniently carry on a regular basis. While well suited for a cosmetic routine that takes place at home, their size and weight makes them less suited for rapid response to a developing blemish or acne sore that is discovered when away from the home. It is believed that a small area plasma device designed for rapid treatment of individual blemishes, also called "spot treatments," will effectively treat a blemish in its early stages and will be portable and convenient.

Direct exposure of a region of biological surface to cold atmospheric plasma may be supplemented with therapy regimes (also referred to as "formulations" or "therapeutic formulations"). For example, plasma generated species, such as ultraviolet photons or reactive oxygen species, may damage biological surfaces. Therefore, therapeutic formulations may be used to prevent or minimize damage to biological surfaces.

In one embodiment, a pre-treatment formulation protects the region against potentially harmful plasma generated species. In another embodiment, a post-treatment formulation neutralizes plasma generated acids that may harm the biological surface following treatment. In one embodiment, plasma-activated media provides effective therapy when applied to the region without direct exposure to the plasma. Without being bound to theory, it is believed that cold atmospheric plasma produces reactive species and secondary reaction products that remain in liquid media following exposure to the plasma.

While a basic plasma treatment is thought to beneficially impact biological systems, the risks of over-exposure or inadvertent damage are non-negligible. Accordingly, conventional plasma devices limit a consumer's ability to implement customized treatment. These treatment risks may be mitigated by customizing the plasma for a specific therapeutic result being sought. In some embodiments, a size of the target treatment area is selectable. In other embodiments, a chemical intermediary may modify the concentrations of potentially harmful species generated in the plasma.

In practice, surface conditions and plasma parameters are coupled, where variation in one induces changes in the other. A sudden shift in surface moisture, for example, may affect electrical conductivity of the surface and lead to an increase in plasma intensity. Conversely, a sudden increase in plasma intensity may vaporize moisture from the surface, in turn changing the properties of plasma. This variability and multi-parameter coupling necessitates control of the plasma treatment device.

Complex interactions between light emission from the plasma, plasma generated species, and biological chemicals native to biological surfaces further complicates cold plasma therapy. In some cases, plasma generated species may acidify a biological surface, thereby aggravating preexisting conditions and outweighing any beneficial outcomes of plasma treatment, for example by light emission, or by exposure to plasma generated species that stimulate wound healing or that would otherwise denature harmful bacteria present in the biological surface. However, measurement of the plasma treatment on a biological surface may allow modulation of the plasma, therefore making the treatment more effective and safer.

Uniformity and steady application of cold plasma during treatment is desirable for several reasons. Non-limiting examples include providing a predictable dose of plasma generated species to the biological surface, providing a consistent treatment result despite variability in conditions of the biological surface, maintaining a uniform exposure to the cold plasma across a region of a biological surface, etc.

### Cold Plasma Therapy Devices

Non-thermal "cold" atmospheric plasma can interact with living tissue and cells during therapeutic treatment in multiple ways. Among the possible applications, cold atmospheric plasma may be used in biology and medicine for sterilization, disinfection, decontamination, and plasma-mediated wound healing.

Several commercialized devices are certified for medical treatment at the present time. These devices are not designed for home use by consumers. Instead, they are designed for use by medical technicians with expertise and training in medical treatment techniques. An example of such device is Rhytec Portrait^{®}, which is a plasma jet tool for topical dermatological treatments. This device features complex power supplies with tightly regulated parameters, using radio-frequency power sources. In addition, the Bovie J-Plasma^{®}, the Canady Helios Cold Plasma, and the Hybrid PlasmaTM Scalpel are all available for use as medical treatment devices. In Germany, the kINPen^{®}, also a plasma jet device, and the PlasmaDerm^{®}, a dielectric barrier discharge (DBD) device, are both certified medical devices that have been introduced to the market within recent years. These devices aim at medical treatment of human tissues, either externally, as in the PlasmaDerm^{®}, or internally. In contrast with the plasma devices for the medical use, the devices for the cosmetic use are geared for a generally intuitive use by consumers, resulting in cosmetic care and pleasant sensation, as opposed to well controlled and certifiable therapeutic effect.

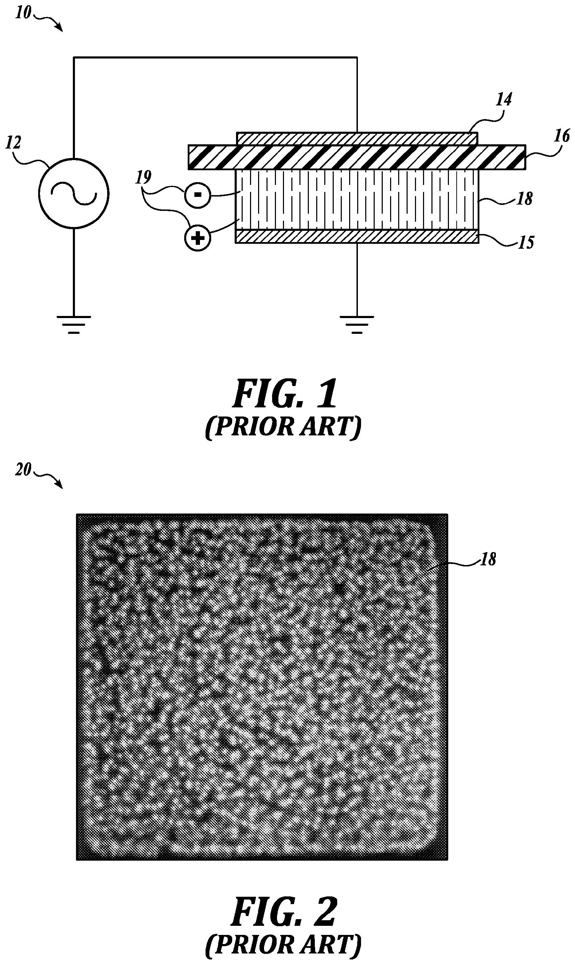

FIG. 1 is a schematic diagram of a plasma generator 10 in accordance with prior art. As shown in FIG. 1, a cold plasma 18 forms through disparate excitation of electrons in a plasma gas by electric fields, relative to the milder excitation effect of the fields on the more massive nuclei of the plasma gas. The cold plasma 18 is formed between a live electrode 14 and a ground electrode 15, also called a counter-electrode, when the live electrode 14 is energized relative to the ground electrode 15 by a power source 12. The power source 12 is an alternating current source or an amplitude modulated direct current source. The cold plasma 18 is a dielectric barrier discharge if the plasma generator 10 includes a dielectric barrier 16 that is placed against the live electrode 14. The cold plasma 18 contains both high temperature electrons 19 and low temperature ions 19 and neutral species. In conventional systems, the plasma gas includes noble gases like helium or argon, and also oxygen and nitrogen containing gases to form reactive oxygen and nitrogen species (RONS). In some cases, as with the PlasmaDerm^{®}, the plasma forms directly in air.

FIG. 2 is an image of dielectric barrier discharges 20 in operation in accordance with prior art. FIG. 2 was obtained as a plan view through a transparent electrode. The plasma 18 forms as multiple discrete filamentary discharges that individually form conductive bridges for ions and electrons 19 to migrate between the electrodes.

For topical treatment, several forms of plasma are used. The first is the gas jet plasma which provides a jet of ions and reactive species that can be directed to a target over varying distances, typically at distances greater than a few millimeters. The medical plasmas described in a preceding paragraph typically feature a gas jet plasma. A second form is the Floating Electrode Dielectric Barrier Discharge (FE-DBD) devices, in which the target substrate (often the human body) acts as a floating ground electrode. The third form is a DBD plasma wand, where the dielectric barrier is placed against a floating ground, instead of the live electrode, and may take the form of a fluorescent tube. The fourth form is a coordinated plurality of dielectric barrier discharge sources. In such an arrangement, a number of atmospheric FE-DBD plasma sources are incorporated into a handheld or flexible device, that is then used to treat one or more anatomical regions.

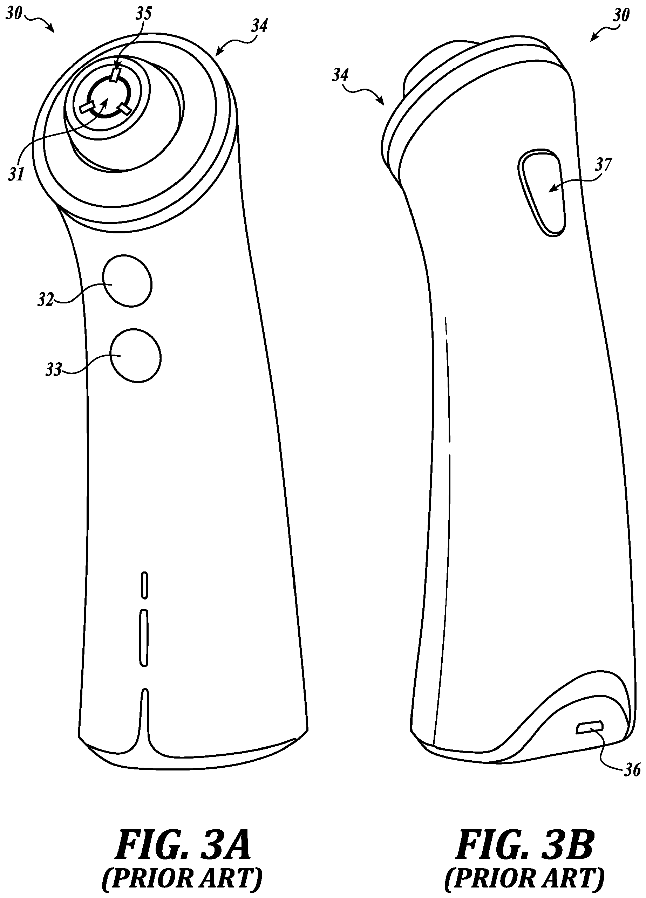

FIGs. 3A and 3B are two views of a cold plasma system in accordance with prior art. A skin treatment device 30 produces cold plasma 18 through a unitary structure that includes a head 31 and a body 34. The device includes one or more user controls, including a plasma power switch 32, and a light switch 33. The head 31 includes one or more light emitting diodes 35 (LEDs). The skin treatment device 30 further includes a plasma pulse control 37, configured to create the plasma 18 at the head 31 while the plasma pulse control 37 is pressed. The skin treatment device 30 includes a charging port 36 for charging an enclosed battery. The skin treatment device 30 includes internal electronic components that drive the plasma 18.

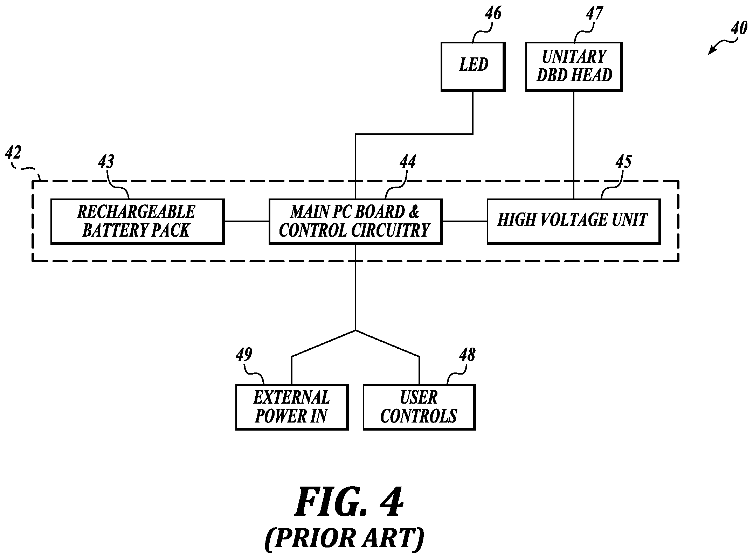

FIG. 4 is a block diagram of a cold plasma system in accordance with prior art. Electronic components 40 include a unitary structure having a DBD head 47 and body 42. The cold plasma 18 is produced between electrodes included in the DBD head 47, which serves as the treatment site. The DBD head 47 is electrically connected to a high voltage unit 45, providing power to the DBD head 47. The power needed to drive the plasma 18 is provided by a rechargeable battery pack 43 enclosed within the body 42. The system includes one or more LEDs 46, connected to the system through a main PC board and control circuitry 44. The main PC board and control circuitry 44 controls the flow of electricity to the LED 46 and the high voltage unit 45, and receives input from one or more user controls 48 and external power in 49 to charge the rechargeable battery pack 43.

Without being bound to theory, it is believed that the effect of cold atmospheric plasma therapy is due to some extent to interaction between RONS and biological systems. A non-exhaustive list of RONS includes: hydroxyl (OH), atomic oxygen (O), singlet delta oxygen (O₂(¹Δ)), superoxide (O₂⁻), hydrogen peroxide (H₂O₂), and nitric oxide (NO). Hydroxyl radical attack is believed to result in peroxidation of cell membrane lipids, in turn affecting cell-cell interaction, regulation of membrane-protein expression, and many other cellular processes. Hydrogen peroxide is a strong oxidizer, believed to have a harmful effect on biological systems. Nitric oxide is believed to play a role in cell-cell signaling and bio-regulation. At the cellular level, nitric oxide is believed to affect regulation of immune deficiencies, cell proliferation, phagocytosis, collagen synthesis, and angiogenesis. At the system level, nitric oxide is a potent vasodilator.

Cold atmospheric plasmas also expose biological surfaces to electric fields, on the order of 1-10 kV/cm. It is believed that cells respond to such fields by opening transmembrane pores. Such electric-field induced cellular electroporation is believed to play a role in transfusion of molecules across cell membranes. Without being bound to theory, the efficacy of treatment is believed to be due at least in part to long-lived plasma-generated species, which in an air plasma will be a variety of RONS at concentrations particular to the operating parameters of the cold atmospheric plasma source.

While cold atmospheric plasma can also be used to ablate tissue or effect treatment in a very short time when operated at high power and intensity, such treatment is believed to harm surrounding tissue and to penetrate far beyond the treated area. Without being bound to theory, it is believed that cold atmospheric plasma treatment at low intensity avoids damaging cells.

Without being bound to theory, it is believed that an important parameter both for direct cold atmospheric plasma treatment and for indirect treatment using plasma-treated media is the dose of plasma species imparted to the treatment surface. In general, this is expressed as a concentration of a given plasma species produced by the cold atmospheric plasma source that is imparted to a unit area of the treated surface over a unit time.

Alternatively, the dose may be expressed as a simple length of time, if the treatment has been determined and the behavior of the cold atmospheric plasma source is well understood. For example, for a stable cold atmospheric plasma source and a uniform surface, a particular dose of a given RONS will be achieved after the cold atmospheric plasma has treated the uniform surface for a given length of time. In practice, surface conditions and plasma characteristics are coupled, where variation in one induces changes in the other. A sudden shift in surface moisture, for example, may affect the conductivity of the surface and lead to an increase in plasma intensity. Conversely, a sudden increase in plasma intensity may vaporize moisture from the surface, producing RONS and changes in the surface. This variability necessitates control of the plasma treatment device, as discussed in greater detail below.

Without being bound to theory, it is believed that cold atmospheric plasma treatment penetrates into the treatment surface through a synergistic effect of electroporation, permeability of plasma generated species, and cell-to-cell signaling. The so called "bystander effect" is thought to play a role in propagating plasma induced cellular changes away from the treatment surface and into a volume beneath it. The bystander effect is believed to occur through chemical signals passed between cells in response to the introduction of a biologically active chemical, potentially amplifying the magnitude of the treatment impact.

In experiments it has been shown that RONS include reactive nitrogen species (RNS) and reactive oxygen species (ROS) that are believed to interact in differing ways to diverse biological surfaces. In agarose films, for example, RONS permeate a volume beneath the film, while in living tissues, only RNS will do so. ROS do penetrate, however, into gelatin and other liquids. ROS, being more reactive than RNS are shorter-lived and are believed to be linked in some circumstances to aggressive or harmful effects on biological surfaces, as previously discussed with respect to hydrogen peroxide.

### Cold Plasma Treatment with Additional Treatment Devices

In some embodiments, a cold plasma system for treating a region of a biological surface includes a plasma treatment device, including an electrode and a dielectric barrier having a first side that faces the electrode and a second side that faces away from the electrode. The system may include an auxiliary treatment device configured to enhance effects of the cold plasma on the region. In an aspect, the auxiliary treatment device is selected from a group consisting of a vibration device, a light source configured to illuminate the region, and a source of air directed to the region.

In an aspect the vibration device includes a multi-axis eccentric mass vibrator. The vibration device may include a piezo-electric actuator.

In an aspect, the light source includes one or more light-emitting diodes, configured to direct light having a wavelength in a range of 400-500 nm toward the region.

In an aspect the source of air includes a conduit configured to direct a stream of air toward the region, an air mover disposed within the conduit, and one or more temperature control elements configured to regulate temperature of the stream of air. The air mover may include a fan. The temperature control elements may include at least one of a thermal-resistive heater coil and a Peltier cooler.

In an aspect, the plasma treatment device further includes one or more actuating members, configured to repeatedly strain and relax the biological surface in the region.

In an aspect, the plasma treatment device further includes a cover placed over the dielectric barrier, and wherein the cover is configured to protect the dielectric barrier from contact damage or contamination. The cover may include glass, plastic, or quartz.

In an aspect, the biological surface includes at least one of: skin, hair, and fingernails.

In an aspect, the plasma is discharged at least partially into the biological surface.

In some examples not according to the invention but present for illustration purposes only, a method of treating a region of a biological surface with a cold plasma system includes generating the plasma by a plasma treatment device that comprises a plasma generator. In some examples not according to the invention but present for illustration purposes only, the method includes applying the plasma to the biological surface, activating an auxiliary treatment device, treating the biological surface with the auxiliary treatment device, and turning off the plasma.

In an aspect, treating the biological surface with the auxiliary treatment device includes vibrating the plasma treatment device.

In an aspect, treating the biological surface with the auxiliary treatment device includes vibrating the biological surface at or near the region.

In an aspect, treating the biological surface with the auxiliary treatment device includes irradiating the region with light from a source of light disposed on the plasma treatment device. The source of light may emit visible light. The source of light may emit infrared light.

In an example not according to the invention but present for illustration purposes only, treating the biological surface with the second treatment device includes providing a stream of flowing air to the region via a conduit disposed within the plasma treatment device and an air mover disposed within the conduit. In an example not according to the invention but present for illustration purposes only, the method includes cooling or heating the stream of flowing air with one or more temperature control elements configured in the conduit.

### Cold Plasma Treatment System with External Support Devices

In some embodiments, a cold plasma system for treating a region of a biological surface includes a plasma treatment device, including an electrode and a dielectric barrier having a first side that faces the electrode and a second side that faces away from the electrode. In an aspect, the plasma treatment device further includes a rechargeable battery electrically connected to the electrode and operably coupled to the power cell via a charging link.

In some embodiments, the plasma treatment system further includes a support device external to the plasma treatment device. In some embodiments, the support device includes a power cell electrically connected to the plasma treatment device and a controller operably coupled to the power cell and to the plasma treatment device. In an aspect, the support device is wirelessly connected to the plasma treatment device. In an aspect, the support device is electrically connected to the plasma treatment device via a detachable cable.

In an aspect, the dielectric barrier and the electrode are disposed on a retractable support, the extension of which positions the second side of the dielectric barrier for treatment of the region, the retractable support being disposed within a housing. In an aspect, the plasma treatment device is a lipstick-size device.

In an aspect, the support device is a smart device. The smart device may be selected from a group consisting of: a smart phone, a tablet, a laptop, an electronic hair-styling device, and a plasma device including a charging dock.

In an aspect, the plasma is discharged at least partially into the biological surface.

In an aspect, the biological surface includes at least one of: skin, hair, or fingernails.

In some embodiments, a cold atmospheric plasma system for treating a region of a biological surface includes a plasma treatment device. The plasma treatment device may include an electrode and a dielectric barrier having a first side that faces the electrode and a second side that faces away from the electrode. In some embodiments, the dielectric barrier and the electrode are disposed on a retractable support the extension of which positions the second side of the dielectric barrier for treatment of the region, the retractable support being disposed within a housing. In some embodiments, the plasma treatment device includes a battery electrically connected to the electrode and a controller operably coupled to the battery and the electrode, configured to receive data and to send control inputs. The system may be a lipstick-sized system.

In some aspects, the battery is a rechargeable battery, enclosed within the device.

In some aspects, the plasma treatment device is configured to connect to a support device external to the plasma treatment device via a detachable cable. The support device may be configured to provide power and control inputs to the plasma treatment device.

In some examples not according to the invention but present for illustration purposes only, a method of treatment of a region of a biological surface with a cold atmospheric plasma system includes attaching a detachable cable to the plasma system and transferring power and control inputs to the plasma system from a support device via the detachable cable. In some examples not according to the invention but present for illustration purposes only, the method further includes generating a cold atmospheric plasma between the plasma system and the region, switching off the cold atmospheric plasma, and detaching the detachable cable from the plasma system.

In some examples not according to the invention but present for illustration purposes only, the method includes extending a retractable support carrying an electrode and a dielectric barrier, the support being disposed within the plasma treatment device, that when extended places the dielectric barrier in position to generate the cold atmospheric plasma in proximity to the region. The method may include retracting the retractable support.

In some examples not according to the invention but present for illustration purposes only, the method includes transferring power wirelessly to the plasma system, wherein the plasma system includes a rechargeable battery.

### Cold Plasma Treatment System with Formulation Dispensing

In some embodiments, a cold plasma system for treating a region of a biological surface, the system includes a plasma generator and a pre-treatment formulation. In some embodiments, the cold plasma system includes an electrode and a dielectric barrier having a first side that faces the electrode and a second side that faces away from the electrode. In an aspect, the system further comprises a post-treatment formulation configured for applying onto the region of the biological surface.

The pre-treatment formulation may be configured for applying onto the region of the biological surface. In an aspect, the pre-treatment formulation comprises plasma treated species. In an aspect, the pre-treatment formulation comprises one or more of a fragrance, an essential oil, a pigment, and an active ingredient.

In an aspect, the biological surface includes at least one of: skin, hair, and fingernails.

In an aspect, the plasma is discharged at least partially into the pre-treatment formulation.

In some examples not according to the invention but present for illustration purposes only, a method of treatment of a region of a biological surface with a cold plasma includes selecting a pre-treatment formulation, applying the pre-treatment formulation to the region, generating a cold plasma between a plasma treatment device and the pre-treatment formulation, and switching off the cold plasma.

In an example not according to the invention but present for illustration purposes only, the method includes removing the pre-treatment formulation from the region. The method may include, prior to applying the pretreatment formulation to the region, treating the pre-treatment formulation by discharging the cold plasma into the pre-treatment formulation.

In an example not according to the invention but present for illustration purposes only, the method includes selecting a post-treatment formulation and, after switching off the cold plasma, applying the post-treatment formulation to the region.

In an example not according to the invention but present for illustration purposes only, the method includes removing the post-treatment formulation from the region.

In some examples not according to the invention but present for illustration purposes only, a method of treatment of a region of a biological surface with a cold-plasma treated formulation includes selecting a formulation, applying the cold plasma to the formulation, switching off the cold plasma, and applying the formulation to the region.

In an example not according to the invention but present for illustration purposes only, the method includes removing the formulation.

In an example not according to the invention but present for illustration purposes only, the method includes, after applying the formulation to the region, discharging the cold plasma into the formulation.

In an example not according to the invention but present for illustration purposes only, the method includes selecting a post-treatment formulation and applying the post-treatment formulation to the region, following application of the formulation. The method may include removing the post-treatment formulation from the region.

### Cold Plasma Treatment with Modular System

In some embodiments, a modular cold atmospheric plasma system for treating a region of a biological surface includes a plasma generator body and a head that is removeably attached to the plasma generator body. The head may have a mounting side facing the generator body and an application side configured to face the biological surface. The application side of the head may carry an electrode and a dielectric barrier having a first side that faces the electrode and a second side that faces away from the electrode.

In some aspects, the plasma is discharged at least partially into the biological surface.

In some aspects, the biological surface includes at least one of: skin, hair, or fingernails.

In some aspects, the head is tapered from a first dimension at the mounting side to a second dimension at the application side. The second dimension may be smaller than the first dimension. In some aspects, the second dimension is larger than the first dimension, and the electrode and the dielectric barrier are configured to produce a cold atmospheric plasma across an enlarged portion of the treatment region on the biological surface with respect to a size of the application side.

In some aspects, the head is a formula-application head that includes a reservoir for a liquid formula and an exuding surface connected to the reservoir via one or more conduits, wherein the exuding surface is configured to allow the liquid formula onto the region.

In some aspects, the head includes a flexible skirt at the application side of the head, and wherein the flexible skirt is configured to conform to the biological surface by compressing the flexible skirt between the biological surface and the head. The flexible skirt may include a rigid spacer that restricts the compression of the flexible skirt between the application side of the head and the biological surface.

In some aspects, the head includes a plasma filter placed between the dielectric barrier and the region. The plasma filter may include at least one of a chemical filter, an ultraviolet filter configured to block the transmission of ultraviolet photons, and a charged species filter that includes a conductive surface configured to neutralize anions, cations, and free electrons. In some aspects, the chemical filter includes at least one of activated carbon, graphene, catalyst, and radical-scavenging material. The plasma filter may be configured to at least partially cover the region of the biological surface.

In some aspects, the application side of the head includes a conformable material at least partially surrounding the dielectric barrier material, wherein the conformable material is conformable to the contours of the region. The electrode and the dielectric barrier may be conformable to the contours of the region.

In some aspects, the head includes one or more air outlets on the application side of the head, configured to provide a cushion of flowing air around the dielectric barrier, and an air mover, enclosed within the air-cushion head, configured to provide the flowing air to the air outlets.

In some aspects, the electrode is pixelated into individually activated areas capable of generating the cold plasma. The system may include a controller configured to energize the activated areas of the electrode.

In some examples not according to the invention but present for illustration purposes only, a method of treatment of a region of a biological surface with a modular cold atmospheric plasma system includes selecting a head from a plurality of removeably attachable heads, attaching the head to a generator body, generating a cold plasma between the plasma system and the region, and switching off the cold plasma.

In some examples not according to the invention, the method includes wherein the head is tapered from a first dimension at a mounting side of the generator body to a second dimension at an application side of the head.

In some examples not according to the invention, wherein the head carries a formula, the method further includes providing the formula at the application side of the head via an exuding opening, and applying the liquid formula to the region of the biological surface.

In some examples not according to the invention, wherein the head is configured to filter the plasma, the method further includes generating the cold plasma through a plasma filter and applying the cold plasma onto the region of the biological surface through the filter.

In some examples not according to the invention, the method includes filtering the plasma through a liquid formula applied to the region of the biological surface.

In some examples not according to the invention, wherein the head is an air-cushion head, the method includes activating an air mover that is located within the head and providing a cushion of flowing air around a dielectric barrier of the head.

In some aspects, the head is a flexible head conformable to the biological surface at the region.

In some aspects, generating the cold atmospheric plasma between the plasma system and the region includes applying a flexible skirt against the region, wherein the flexible skirt surrounds a dielectric barrier of the head and containing the cold atmospheric plasma in a space between the region, the flexible skirt, and the head.

### Cold Plasma Treatment with Sensors and Controlled Plasma Generation

In some embodiments, a cold plasma system for treating a region of a biological surface includes a plasma treatment device, including an electrode and a dielectric barrier having a first side that faces the electrode and a second side that faces away from the electrode. The system may include one or more sensors. The sensors may measure properties of at least one of a cold plasma, the ambient environment around the system, and the biological surface. The system may include a controller operably coupled to the plasma treatment device and to at least one of the sensors. The controller may receive input from the sensors to determine control data for the plasma treatment device and send control data to the plasma treatment device.

In an aspect, the electrode is pixelated into a plurality of areas that are individually addressable by the controller.

In an aspect, the one or more sensors include a sensor placed on or near the region of the biological surface.

In an aspect, the one or more sensors include one or more motion sensors. In an aspect, the one or more sensors include a humidity sensor configured to measure a humidity of ambient air. In an aspect, the one or more sensors include a reactive oxygen sensor. In an aspect, the one or more sensors include a light sensor. In an aspect, the one or more sensors include a plasma conductivity sensor. In an aspect, the one or more sensors include a surface temperature sensor. In an aspect, the one or more sensors include a distance sensor. In an aspect, the one or more sensors include an ion concentration sensor.

In an aspect, the plasma is discharged at least partially into the biological surface.

In an aspect, the biological surface includes at least one of: skin, hair, and fingernails.

In an aspect, the cold plasma system further includes a ballast circuit connected to the electrode.

In an aspect, the cold plasma system further includes a non-transitory computer readable medium having computer executable instructions stored thereon that, in response to execution by one or more processors of a computing device, cause the computing device to perform actions including determining a uniformity of the plasma as a function of time, determining a dose of one or more plasma generated species, and modulating the plasma as a function of time to control the uniformity and the dose.

In an aspect, the cold plasma system further includes a formulation engine that, in communication with the controller, determines a target dose of plasma and a set of plasma parameters necessary for providing an application dose and a data storage system that stores data related to the application dose.

In some examples not according to the invention but present for illustration purposes only, a method of treatment of a region of a biological surface with a cold plasma includes generating the cold plasma between a plasma source and the region and measuring one or more treatment parameters with one or more sensors. In some embodiments, the method includes determining a plasma dose from the treatment parameters, modulating one or more of the treatment parameters to adjust the plasma dose, and switching off the cold plasma.

In an example not according to the invention, the method includes at least one sensor being carried by the plasma source.

In an example not according to the invention, the method includes at least one sensor being carried by the biological surface.

In an example not according to the invention, the method includes moving the plasma source while the plasma source is generating the cold plasma.

In an example not according to the invention, the method includes providing a perceptible signal when the plasma dose is outside a safe range or an effective range.

In an example not according to the invention, the method includes determining a discharge voltage as a function of time, determining a discharge current as a function of time, determining a plasma temperature as a function of time, and determining a gas temperature near the region as a function of time.

In an example not according to the invention, the method includes, prior to generating the plasma, placing at least one of the sensors onto the biological surface at or near the region, exposing the at least one sensor to the plasma, and, after generating the plasma, removing the at least one sensor.

In an example not according to the invention, the method includes automating a determination of a treatment dose by determining the treatment dose via a formulation engine that is in communication with a data storage system, determining a set of plasma parameters for achieving the treatment dose during treatment of the region, and providing the treatment dose and the set of plasma parameters to the plasma source.

In an example not according to the invention, the method includes intermittently redefining the treatment dose by re-measuring the plasma parameters during the cold plasma treatment.

In an example not according to the invention, the method includes determining an indicator of uniformity of the plasma; and modulating one or more of the plasma parameters in response to changes in the indicator.

In an example not according to the invention, the method includes measuring the plasma parameters from a group including a current provided to the discharge, a driving frequency, a voltage waveform, a peak to peak voltage, a root mean square voltage, a plasma temperature, a gas temperature, and optical emission from the plasma.

In an example not according to the invention, the method includes generating the plasma by a plurality of pixelated electrodes arranged in a matrix, and determining a discharge power for a given pixelated electrode. In an example not according to the invention, the method includes modulating the discharge power for the given pixelated electrode if the plasma has localized to the given pixelated electrode.

### DESCRIPTION OF THE DRAWINGS

The foregoing aspects and advantages of the inventive technology will become more readily appreciated as the same become better understood by reference to the following detailed description, when taken in conjunction with the accompanying drawings, wherein:
FIGURE 1 is a schematic diagram of a plasma generator in accordance with prior art;
FIGURE 2 is an image of a dielectric barrier discharge surface in operation in accordance with prior art;
FIGURES 3A-3B are two views of a cold plasma system in accordance with prior art;
FIGURE 4 is a block diagram of a cold plasma system in accordance with prior art;
FIGURE 5 is a schematic diagram of a cold plasma treatment system in accordance with the present disclosure;
FIGURE 5A is a schematic diagram of a cold plasma treatment system in accordance with the present disclosure;
FIGURE 6 is a schematic diagram of a cold plasma treatment system in accordance with the present disclosure;
FIGURE 7 is a schematic diagram of a cold plasma treatment system in accordance with the present disclosure;
FIGURE 8 is a schematic diagram of a cold plasma treatment system in accordance with the present disclosure;
FIGURE 9 is a schematic diagram of a cold plasma treatment system in accordance with the present disclosure;
FIGURE 9A is a schematic diagram of a cold plasma treatment system in accordance with the present disclosure;
FIGURE 10 is a flowchart of a method of cold plasma treatment not according to the invention but present for illustration purposes only;
FIGURE 11 is a schematic diagram of a cold plasma treatment system in accordance with the present disclosure;
FIGURE 11A is a schematic diagram of a cold plasma treatment system in accordance with the present disclosure;
FIGURE 11B is a schematic diagram of a cold plasma treatment system in accordance with the present disclosure;
FIGURE 11C is a schematic diagram of a cold plasma treatment system in accordance with the present disclosure;
FIGURE 11D is a schematic diagram of a cold plasma treatment system in accordance with the present disclosure;
FIGURE 12 is a schematic diagram of a cold plasma treatment system in accordance with the present disclosure;
FIGURE 13 is a schematic diagram of a cold plasma electrode system in accordance with the present disclosure; and
FIGURE 14 is a flowchart of a method of cold plasma treatment not according to the invention but present for illustration purposes only.

### DETAILED DESCRIPTION

While several embodiments have been illustrated and described, it will be appreciated that various changes can be made therein without departing from the spirit scope of the invention. The invention is defined by independent claim 1. Preferred embodiments are defined in the dependent claims.

### Cold plasma system with additional treatment devices

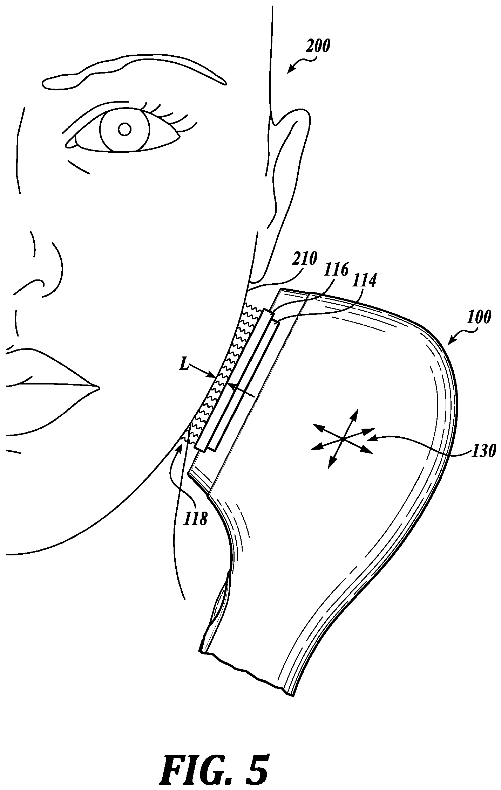

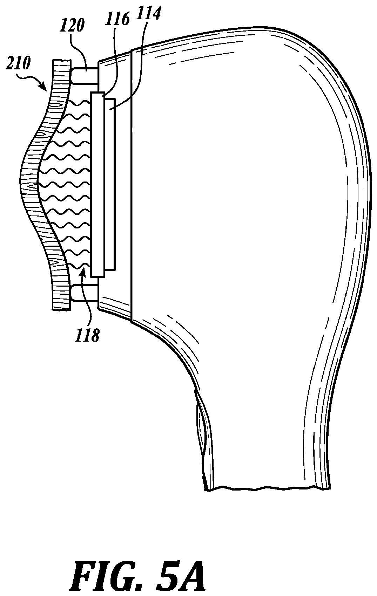

FIG. 5 presents a schematic diagram of a cold plasma treatment system in accordance with the present disclosure. In some embodiments, the cold plasma treatment system provides cosmetic treatment of a region of a biological surface 210 of a consumer 200. In some embodiments, the system includes a cold atmospheric plasma treatment device 100 including a plasma generator having an electrode 114 and a dielectric barrier 116.

In some embodiments, the plasma treatment device 100 includes a vibration device 130. Without being bound to theory, it is believed that the actuation of the vibration device 130 provides the consumer 200 with an enhanced treatment experience, and improves treatment efficacy by mitigating plasma 118 non-uniformity over the region. In some embodiments, the vibration device 130 may emit ultrasound vibrations. In operation, the ultrasound may enhance the transport of active plasma species into the tissue (or across diffusion barriers, in general) therefore increasing the efficacy of cold plasma treatment.

The vibration device 130 may vibrate the treatment device 100, thereby affecting the distance L between the second side of the dielectric barrier 116 and the biological surface 210. In some embodiments, the vibration device 130 vibrates the treatment device 100 in multiple axes simultaneously. In other embodiments, the vibration device 130 vibrates the treatment device 100 along only one axis. The vibration device 130 may vibrate the treatment device 100 such that the plasma 118 moves parallel to the biological surface 210 in one or two axes. It is believed that such movement distributes the plasma 118 across the region, thereby improving plasma 118 uniformity. The vibration device 130 may include one or more vibration sources, such as a piezoelectric actuator or a multi-axis eccentric mass vibrator.

FIG. 5A illustrates a cold plasma treatment system in accordance with the present disclosure. In some embodiments, the plasma treatment device 100 directly actuates the biological surface 210 by one or more actuating members 120. Without being bound to theory, it is believed that repeated tension and compression of the biological surface 210 enhances the efficacy of multimodal treatment by stimulating synergistic effects with permeability of plasma generated species and consumer 200 experience of the treatment. The actuating members 120 may be in direct contact with the biological surface 210 at or near the region. In some embodiments, the actuating members 120 move in opposite directions to each other, parallel to the biological surface 210. The actuating members 120 may move towards each other, in turn compressing and releasing the biological surface 210. The actuating members 120 may move away from each other, in turn stretching and releasing the biological surface 210. In some embodiments, the actuating members 120 move both towards and away from each other, thus both stretching and compressing the biological surface 210. In some embodiments, the plasma 118 is generated toward the region while the actuating members 120 actuate the biological surface 210.

The actuation may be in the form of the ultrasound emitted by the vibration device 130. In different embodiments, the ultrasound may be transmitted to the target biological surface 210 through the actuating members 120 capable of generating ultrasound. In some embodiments, the actuating members 120 form an annular shape with the electrode 114 and the dielectric barrier 116 contained within the annular shape. In other embodiments, a source of ultrasound may include multiple apertures for directing cold plasma toward the biological surface 210. In operation, an acoustic coupling medium may couple the source of ultrasound (e.g., the actuating members 120) with the biological surface 210. Some nonexclusive examples of such coupling media are hydrogels, solid gel pads, etc. The coupling medium can be formulated to have additional ingredients and properties to enhance the experience, such as precursors and ingredients that can be activated by plasma and/or work with it, fragrance etc. In some embodiments, no coupling gel is used and the source of ultrasound couples enough energy to biological surface even in the absence of coupling medium.

The actuating members 120 may actuate the surface without plasma 118 exposure, thereby providing a tactile experience to the consumer 200.

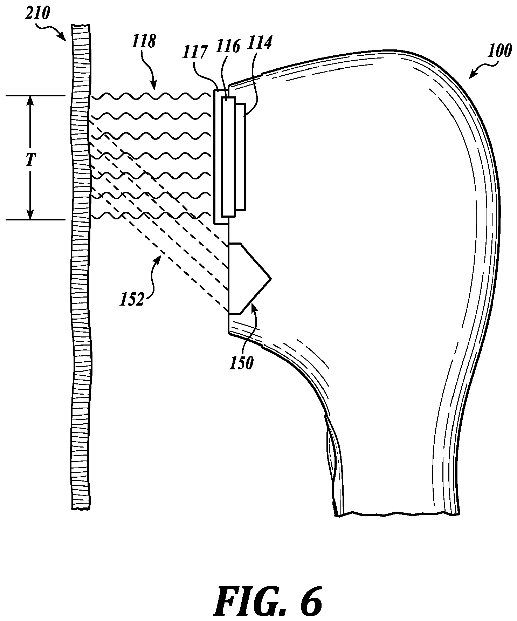

FIG. 6 is a schematic diagram of a cold plasma treatment system in accordance with the present disclosure. In addition to treatment by the plasma 118, the plasma treatment device 100 may include a light source 150, configured to illuminate the region with light 152 within the area described by the characteristic dimension T. As previously described, it is believed that irradiation of the biological surface 210 with light having a wavelength in the range of 400-500 nm provides desirable therapeutic results for cosmetic treatment of blemishes. In some embodiments, the plasma treatment device 100 includes multiple light sources. The light source 150 may include one or more light emitting diodes, individually emitting light having a wavelength within a target range.

The light source 150 may include an infrared light element, providing radiative heating to the biological surface 210. Without being bound to theory, it is believed that radiative heating of the biological surface enhances the therapeutic effect of plasma treatment by triggering a response of the biological surface 210 to plasma generated species and by providing an enhanced experience for the consumer 200.

The plasma treatment device 100 may include a cover 117 disposed on or over the dielectric barrier 116. Non-exclusively, the cover 117 may include plastic, glass, or quartz, and may block plasma generated species from reaching the biological surface 210. Without being bound to theory, it is believed that the plasma 118 may emit ultraviolet photons under certain conditions. As such, it may be desirable to block the transmission of ultraviolet photons using a cover 117.

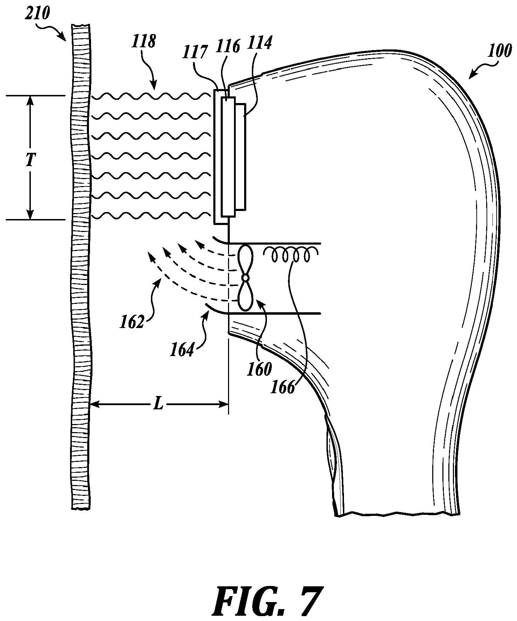

FIG. 7 is a schematic diagram of a cold plasma treatment system in accordance with the present disclosure. In some embodiments, the plasma treatment device 100 includes a source of air that directs an air stream 162 to the region within the area described by the characteristic dimension T. The source of air may include an air mover 160, such as a fan or a blower, disposed within an air conduit 164 that is shaped to provide the air stream 162 at the surface of the region. In some embodiments, one or more temperature control elements 168 disposed within the plasma treatment device 100 adjust the temperature of the air. Non-limiting examples of the temperature control elements 168 include thermoelectric cooling elements including Peltier coolers, electric heating elements including resistive heating coils, etc. In some embodiments, a volatile oil is disposed within the air conduit 164 that contains a fragrance such that, when the air mover 160 is active, the oil imparts a pleasant aroma to the region.

### Small Size Device

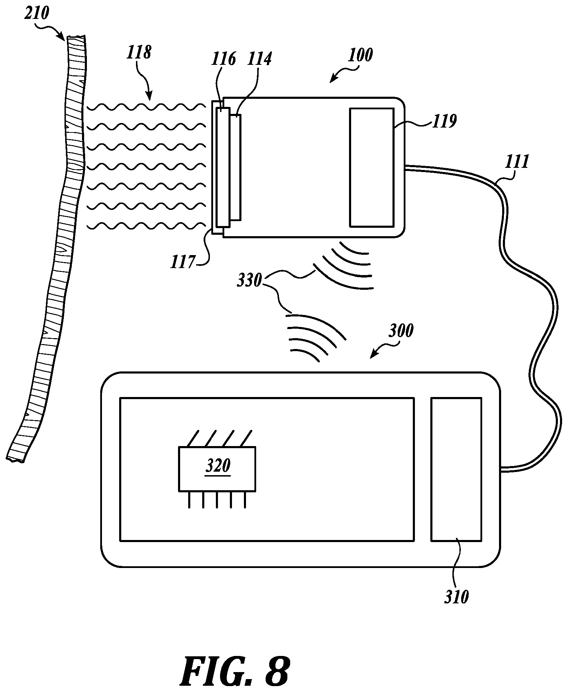

FIG. 8 presents a schematic diagram of a cold plasma treatment system in accordance with the present disclosure. In some embodiments, the plasma treatment device 100 is electrically connected to an external device 300 having a power cell 310 and a controller 320. In some embodiments, the plasma treatment device 100 is electrically connected to the external device via a cable 111. In some embodiments, the cable 111 carries control inputs and electrical power to the plasma treatment device 100. In some embodiments, the cable 111 is detachable from the plasma treatment device 100, the external device 300, or both. The power cell 310 may be a rechargeable battery including, for example a lithium ion battery. The controller 320 may be capable of receiving data and sending control signals to the plasma treatment device 100.

In some embodiments, the plasma treatment device 100 includes a battery 119 electrically connected to the electrode 114. The battery 119 may be rechargeable, charged by connecting the cable 111 to the plasma treatment device 100 and to a power source. Some non-limiting examples of such power source are the external device 300, an adapter connected to a standard wall outlet providing electricity, a solar cell, etc. In some embodiments, the battery 119 charges wirelessly 330. In some embodiments, the battery 119 is a commercially available battery, such as a battery of one of the A-series types ("A," "AA," or "AAA").

In some embodiments, the external device 300 is a smart phone. In some embodiments, the external device 300 is a laptop or a tablet, configured to be compatible with the plasma treatment device 100 and to provide power and control inputs to the external device 300. In some embodiments, the external device 300 is a cosmetic tool, including but not limited to an electronic beard trimmer, a hair iron, a hair drier, an electronic epilator, etc. The external device 300 may be a large area plasma treatment device, as described previously, further including a charging dock for electrically connecting to the plasma treatment device 100. In some embodiments, the charging dock is configured to accept the plasma treatment device 100, which can be operably mounted into the large-area device for compact charging and operation as a plasma generator.

In some embodiments, the electrode 114 and the dielectric barrier 116 are disposed behind a cover 117. The cover 117 may be removable. The cover 117 may provide protection for the dielectric barrier 116 when the plasma treatment device 100 is not in use.

In some embodiments, the electrode 114 and the dielectric barrier 116 are disposed on a retractable support enclosed within the plasma treatment device 100. The retractable support may be configured such that when retracted, the dielectric barrier 116 and the electrode 114 are hidden from view and the plasma treatment device 100 cannot be activated. The retractable support may rotate through the action of a mechanism disposed at an end of the plasma treatment device 100 opposite to the dielectric barrier 116, such that the dielectric barrier 116 emerges from the opposite end of the plasma treatment device 100 in a manner resembling a lipstick. The plasma treatment device 100 may have a form factor similar or comparable to a retractable lipstick tube, such that it resembles the lipstick tube when inactive. In some embodiments, the retractable support is a linear slide that is configured to slide the electrode 114 and the dielectric barrier 116 behind the shield 119 when not in use.

In some embodiments, the plasma treatment device 100 is controlled via a user interface in the external device 300. In some embodiments, the external device 300 is any type of device including a battery, a general purpose computer, and computer readable memory with instructions stored thereon that, when executed by the computer implement a method of treatment of a region of a biological surface by cold atmospheric plasma.

In some embodiments, the plasma treatment device 100 includes one or more user controls including, but not limited to, a power switch, a plasma intensity selector, and a safety switch. The plasma treatment device 100 may be switched on and switched off using a power switch disposed on the plasma treatment device 100, and the plasma 118 is generated while the plasma treatment device 100 is on. In some embodiments, a safety switch prevents the plasma treatment device 100 from turning on until the safety switch is disengaged. In some embodiments, the safety switch is a fingerprint reader. In some embodiments, a plasma intensity selector permits smooth and continuous modulation of the plasma intensity, in terms of a power supplied to the electrode 114. In some embodiments, the plasma intensity selector limits the plasma treatment device 100 to one of a number of discrete intensity settings, in terms of incremental steps in the power supplied to the electrode 114.

In some embodiments, the plasma treatment device 100 includes one or more light emitting diodes (not shown), providing therapeutic light to the biological surface 210. In some embodiments, the light emitting diodes provide blue light, in the range of 400 - 500 nm.

### Cold plasma with formulation dispensing

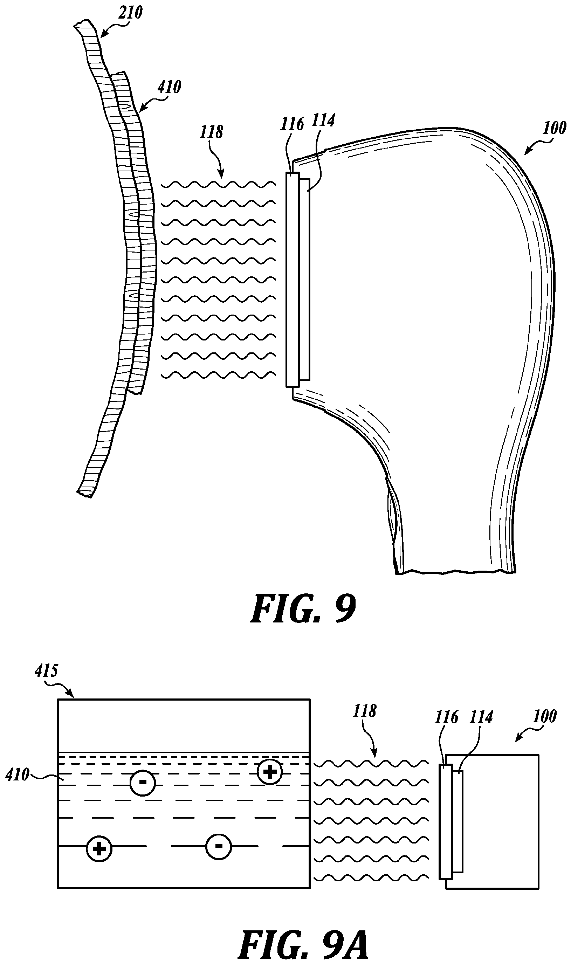

FIG. 9 presents a schematic diagram of a cold plasma treatment system in accordance with the present disclosure. In some embodiments, the plasma treatment device 100, including the dielectric barrier 116 and the electrode 114, discharges the plasma 118 into the biological surface 210 through a formulation 410. The formulation 410 may include one or more active ingredients, including but not limited to anti-oxidants, radical scavenging compounds, ultraviolet absorbing compounds, rejuvenating compounds, etc. In some embodiments, the radical scavenging compound is an anhydrous, glycol-in-silicone formula with ascorbic acid and ascorbyl glucoside. In some embodiments, the radical scavenging compound is a water-in-silicone emulsion with a large internal aqueous phase incorporating water-soluble active ingredients. Without being bound to theory, it is believed that the aqueous phase will form encapsulations, containing active ingredients. Rejuvenating compounds may include collagen, elastin, and the like. The formulation may include inactive ingredients, such as dyes, pigments, fragrances, essential oils, emulsifiers, viscosity modifiers, etc. In some embodiments, the dye may be chemically reactive, and may respond to changes in pH induced by exposure to the plasma 118.

As shown in FIG. 9A, in some embodiments the plasma 118 is discharged into the formulation 410 in a container 415, before application to the biological surface 210 at or near the region. Without being bound to theory, it is believed that the plasma 118 generates beneficial species in the plasma, including ions, radicals, and long-lived RONS. The plasma treatment device 100 may generate the plasma in proximity of the formulation 410, by placing the plasma treatment device 100 near the exposed surface of the formulation 410 while it is in the container 415.

In some embodiments, a pre-treatment formulation enhances the effects of exposure to the plasma 118 by including reagent compounds to generate RONS. In some embodiments, a post-treatment formulation reduces the potentially harmful effects of prolonged exposure to plasma generated species. For example, the post-treatment formulation may control the pH shift of the region after exposure to plasma generated species by including buffer compounds.

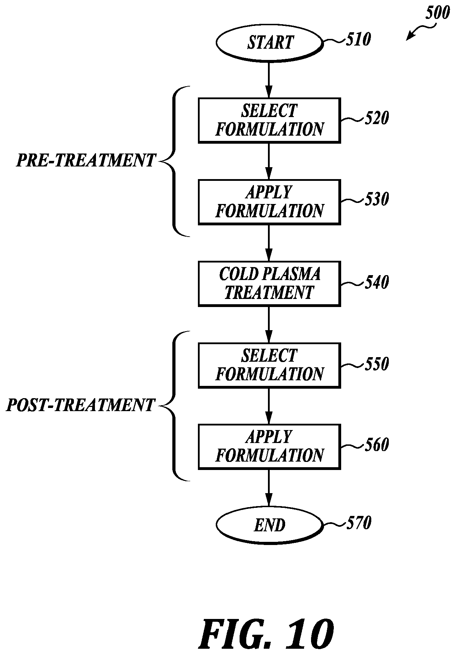

FIG. 10 illustrates a method of treatment 500 using the plasma treatment device 100 to generate the plasma 118 between the plasma treatment device 100 and the biological surface 210 that includes at least one formulation 410. The method is not according to the invention and present for illustration purposes only.

In some embodiments, the method may include additional steps or may be practiced without all steps illustrated in the flow chart.

The method starts in block 510, and proceeds to a pre-treatment phase, including selecting a formulation, as shown in block 520, and applying the formulation to the region, as shown in block 530. As previously described, the formulation 410 may have protective or enhancing properties that improve therapeutic results following exposure to the plasma 118. In some embodiments, the formulation 410 is selected for reducing exposure of the region to ultraviolet photons produced in the plasma, or for enhancing production of RONS, etc.

In some embodiments, a pre-treatment formulation is applied to the region before exposure to the plasma 118. The method then proceeds to block 540, which includes generating the plasma 118. The plasma treatment device 100 may generate the plasma 118 in proximity to the region. The plasma treatment in block 540 may continue until the plasma 118 turns off. In some embodiments, the method then proceeds to block 550, where a post-treatment formulation is selected. The post-treatment formulation may be applied to the region following exposure to the plasma 118. The pre-treatment formulation and the post-treatment formulation may be identical or different, and selected to provide different effects to the region. The method ends in block 570. In some embodiments, the method includes removing the pre-treatment formulation following plasma treatment 540. In some embodiments, the method includes removing the post-treatment formulation after applying the post-treatment formulation 560.

### Modular Cold Plasma Generating Device

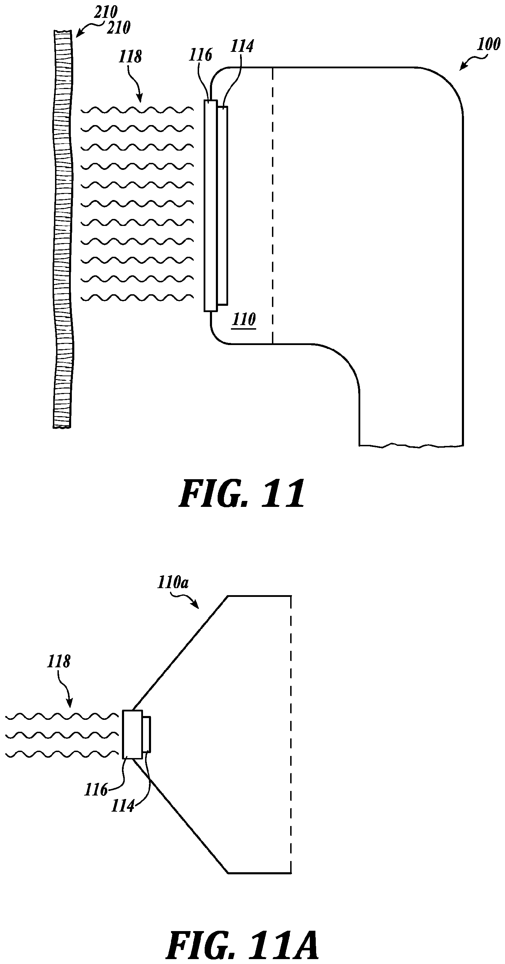

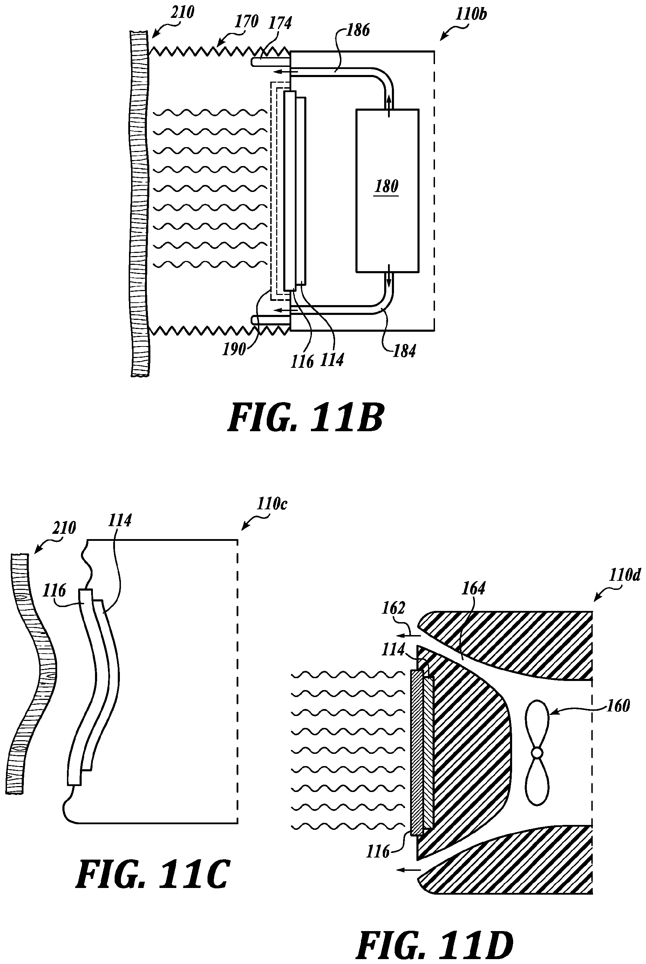

FIG. 11 is a schematic diagram of a cold plasma treatment system 100 in accordance with the present disclosure. In some embodiments, the system includes a treatment device body 100 and a head 110 that is removeably attached to the treatment device body 100. The illustrated head 110 has a mounting side facing the treatment device body 100 and an application side carrying an electrode 114, and a dielectric barrier 116 has a first side facing the electrode 114 and a second side facing away from the electrode 114. The cold plasma system 100 may include a plurality of attachable heads 110 for cosmetic treatment over a region of a biological surface 210. The biological surface 210 includes, but is not limited to, skin, hair, fingernails, etc.

In some embodiments, a head 110-x is selected to produce the cold plasma 118 to execute a particular treatment. For example, when treating a relatively small region on the biological surface 210, a size of plasma 118 may be selected to avoid exposing the non-target portion of the biological surface 210 to plasma-generated species. Here, the term "size of plasma" refers to a characteristic or a descriptive dimension of the plasma. For example, for a plasma generated by a round electrode 114, the characteristic dimension of the plasma is related to a diameter of the electrode 114.

As illustrated in FIG. 11A, a head 110a may be selected and attached to the treatment device body 100. The head 110a tapers from a larger size at the mounting side to a smaller size at the application side. Therefore, the illustrated head 110a generates the plasma 118 having a characteristic size that differs from the diameter of the mounting side of the head 110a. While FIG. 11A illustrates the head 110a having the application side that is smaller than the attachment side, it should be understood that the reverse is also possible. For example, the head 110a may have its application side larger than the attachment side to cause a low intensity treatment over a region of the biological surface 210.

As illustrated in FIG. 11B, a head 110b may include a formula reservoir 180 and an exuding surface 186 on the application side of the head 110b. The exuding surface 186 may be connected to the formula reservoir 180 via one or more conduits 184. In some embodiments, the formula exuding surface 186 includes one or more nozzles on the application side of the head 110b. In some embodiments, the formula exuding surface 186 is a porous material having a void volume to buffer the flow of formula from the formula exuding surface. The porous material may include a cured gel, a soft plastic foam, a rigid plastic foam, a natural porous material such as pumice, etc. In some embodiments, the formula exuding surface 186 may include a vent barred by one or more grills, a wire mesh screen, a patterned perforated screen, etc.

In some embodiments, the formula reservoir 180 is compressed by pressure when the application side of the head 110b is applied to the biological surface 210. In some embodiments, the formula reservoir 180 is compressed by a mechanism enclosed within the head 110b including, but not limited to an electric actuator, a servo, a manually operated lever, a roller, a pair of rollers, etc. In some embodiments, the formula reservoir 180 is removable and interchangeable, and contains a prepared formula tailored to a desired therapeutic or cosmetic result.

In some embodiments, the formula includes one or more cosmetic ingredients. Cosmetic ingredients may include a fragrance, a pigment, a cream, an oil, a natural extract, a moisturizer, etc. In some embodiments, the formula includes one or more medicaments, for example, astringents, pharmaceutically active compounds, acid neutralizing creams, anti-oxidants, etc. In some embodiments, the formula includes one or more protective compounds to protect the biological surface from potentially harmful effects of exposure to the plasma 118. Some non-limiting examples of such protective compounds are an anti-oxidant, a moisturizer, a clarifying cream, an acidity buffering cream, etc.

In one embodiment, the head 110b includes a flexible skirt 170 at the application side of the head 110b. In some embodiments, the flexible skirt 170 is made from corrugated plastic or soft rubber, and attached to the application side of the head 110b. In some embodiments, the flexible skirt 170 is compressed by contacting the biological surface 210. In some embodiments the flexible skirt 170 includes a rigid spacer 174, restricting the compression of the skirt 170, thereby defining a minimum spacing between the head 110b and the biological surface 210. In some embodiments the flexible skirt 170 is impermeable to gases and, when compressed, creates a contained environment for the plasma 118 to form therein. The rigid spacer 174 may be enclosed by the flexible skirt 170 or may be external to it, and may be added or removed. In some embodiments, the rigid spacer 174 includes a conductive material including but not limited to a metal. In some embodiments, the rigid spacer 174 including a conductive material is biased at a voltage greater than or equal to zero. Without being bound to theory, it is believed that the rigid spacer 174 thus biased may allow the plasma to form between the head 110b and the rigid spacer 174, thereby reducing the dose of ions and electrons directed to the biological surface 210. In some embodiments, the plasma 118 discharging into the rigid spacer 174 produces RONS that are contained in the volume defined by the flexible skirt 170.

In one embodiment, the head 110b includes a filter 190 for filtering the plasma 118. The filter 190 may be placed between the head 110b and the biological surface 210, e.g., on a path of the plasma 118 applied to the biological surface.

In some embodiments, the filter 190 is an ultraviolet filter, placed at least partially to block the path of ultraviolet photons from the plasma 118 to the biological surface 210. In some embodiments, the filter 190 blocks ultraviolet photons because the filter is made of UV absorbent or UV scattering material, including, but not limited to, plastic, glass or quartz treated with a UV-blocking film, etc.

In some embodiments, the filter 190 is a chemical filter designed to sequester or convert one or more plasma generated species that would otherwise reach the biological surface 210. In some embodiments, the filter 190 includes a carbonaceous material, non-limiting examples of which include graphene, carbon nanotubes, activated carbon paper, carbon fiber, etc. In another embodiment, the filter 190 includes a catalytic material, non-limiting examples of which include metal particles embedded in a porous matrix. In some embodiments, the filter 190 includes radical scavenging materials, for example, antioxidants, including catalases, glutathione peroxidase, superoxide dismutase (SOD), α-tocopherol (Vit. E), ascorbic acid (Vit. C), β carotene (Vit. A), selenium, etc. In some embodiments, the filter includes a pH sensitive polymer that responds to changes in proton concentration by changing its porosity, surface properties, dimensions, etc. Some non-limiting examples of such pH sensitive polymers include polyacids and polybases, chitosan, hyaluronic acid, and dextran. In some embodiments, the filter responds to changes in pH by opening pores and releasing one or more of the previously described radical scavenging materials.

In some embodiments, the filter 190 includes a liquid formula that is applied to the biological surface 210 upon contact. The liquid formula may include any of the previously mentioned filter materials, carried in a liquid emulsion including but not limited to a cream or an oil. In some embodiments, the liquid formula filter 190 includes additional materials such as cosmetic ingredients, medical ingredients, etc. In some embodiments, the liquid formula includes an indicator material that provides a colorimetric indicator of exposure to plasma generated species. In some embodiments, the indicator material is a pH sensitive dye that will change color when the biological surface 210 has been exposed to a concentration of plasma-generated acidifying or alkalizing species that is sufficient to alter the molecular structure of the dye. Non limiting examples of pH sensitive dye include Gentian violet, Methyl yellow, Methyl red, Cresolphthalein, Indigo carmine, etc.

In some embodiments, the filter 190 includes a charged particle filter placed between the plasma 118 and the biological surface 210 that attracts and neutralizes charged particles present in the plasma 118. In some embodiments, the charged particle filter includes one or more conductive elements, individually biased at a nonzero voltage. Non-limiting examples of a conductive element include a metal screen, a metal probe, a metal ring, etc., placed near or around the dielectric material 116 on the application side of the head 110b. In some embodiments, the charged particle filter selectively filters out positive ions by having a negative polarity, therefore neutralizing the positive ions that approach the surface of the filter 190. In some embodiments, the charged particle filter filters out all charged particles by combining multiple conductive elements, e.g., at least one conductive element carrying a negative polarity and at least one conductive element carrying a positive polarity.

As illustrated in FIG.11C, the biological surface 210 includes contours that may affect the uniformity of exposure of the region to the plasma 118. Non-limiting examples of contoured biological surfaces 210 include regions on a face and body, including but not limited to convex surfaces such as the cheekbones, the chin, the eyebrows, the nose, the jaw, knuckles, ankles, elbows, knees, etc. Similarly, contoured biological surfaces 210 may include concave surfaces, as in the area beneath the jaw, around the ears, along the neck, etc. In some embodiments, a head 110c includes a conformable material on the application side. The conformable material is configured to reversibly conform to the contours of the region. Non-limiting examples of the conformable material include gel, cured foam, rubber, plastic, etc. In some embodiments, the conformable material on the head 110c includes a consumable material, for example a dry solid, a moisturizing gel, a water soluble cream, etc.

In some embodiments, the application side of the head 110c is reversibly conformable with respect to the biological surface 210. In some embodiments, the dielectric barrier 116 includes a flexible surface, including but not limited to a woven dielectric cloth, such as a glass cloth, a ceramic cloth, etc. In some embodiments, the electrode 114 includes a flexible conductive surface, such as a woven metal cloth, copper mesh, stainless steel mesh, etc. In some embodiments, the flexible surface included in the dielectric barrier 116 is sealed to prevent accumulation of material abraded from the biological surface 210 during the plasma treatment. The flexible surface may be sealed with a coating including, but not limited to, Teflon, SiOₓ film, graphene, etc.

As illustrated in FIG.11D, a head 110d may provide an air cushion between the head 110d and the biological surface 210. In some embodiments, the head 110d includes a plurality of air conduits 164 that at least partially surround the electrode 114 and the dielectric barrier 116. In operation, the air mover 160 provides air to the air conduits 164 (e.g., nozzles, vents, etc.) that direct a vectored flow of air away from the head 110d. The flow of air may create an air cushion that prevents or at least minimizes a contact between the head 110d and the biological surface 210. In some embodiments, the air mover160 is an electric fan, located within the head 110d. The air mover may operate independently from the electrode 116 and may be turned on and turned off without altering the state of the plasma 118.

### Cold Plasma Device with Sensors

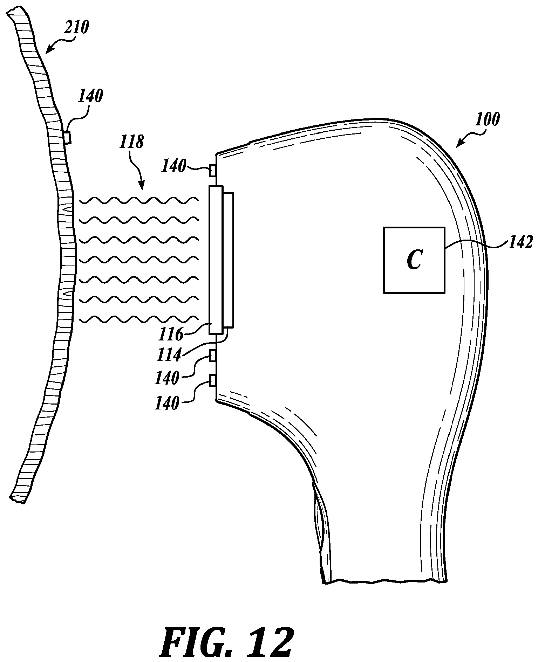

FIG. 12 is a schematic diagram of a cold plasma treatment system in accordance with the present disclosure. In some embodiments, the cold plasma treatment device 100 includes one or more sensors 140 to measure plasma parameters. Based on the measured plasma parameters, a controller 142 may control the cold atmospheric plasma 118 and maintain a predetermined cosmetic treatment over a region of a biological surface 210.

As previously described, in some embodiments, the cold atmospheric plasma 118 is formed using the biological surface 210 as a floating reference electrode. Without being bound to theory, it is believed that such an arrangement is sensitive to non-uniform distribution of water and ion concentrations over the biological surface 210. It is believed that a localized region that is relatively rich in ions, such as a sweat gland, may provide a preferred conductive path for plasma-generated charged species, and the cold atmospheric plasma 118 may form preferentially at such a site on the biological surface 210. In turn, plasma preference for a particular location over another on a biological surface 210 introduces poorly controlled non-uniformity in treatment and variability in plasma dosage over the region treated by the plasma 118. It is believed that uniformity is an important criterion in the operation of a cold atmospheric plasma source. Therefore, in at least some embodiments, the design of the plasma treatment device 100 takes into account the sensitivity of the cold atmospheric plasma 118 to variations in properties of the surface 210.

Uniformity of the plasma 118 is defined in terms of a variability of one or more plasma parameters, for example, discharge power, discharge volume, the concentrations of plasma generated species, etc. In a highly variable system, for example, where the treatment region contains many discrete sub-regions of disparate properties, the plasma treatment device 100 may exhibit discontinuities in the discharge current or discharge voltage as the plasma treatment device 100 translates between ion-rich and ion-poor sub-regions of the surface210. Without being bound to theory, it is believed that a plasma source, passing over a conductive sub-region may exhibit a spike in discharge current and a corresponding drop in discharge voltage.

In some embodiments, the controller 142 actuates an electronic ballast circuit, connected to the electrode 114. Without being bound to theory, it is believed that an electronic ballast circuit may permit the controller 142 to regulate the current to electrode 142, thereby preventing thermal runaway of the plasma 118 and constriction of the plasma 118 at one or more localized spots on the biological surface 210.

As illustrated in FIG. 12, which demonstrates an embodiment of the inventive technology, the plasma source 100 incorporates one or more sensors 140 to measure parameters of a cold atmospheric plasma 118 and a biological surface 210. In some embodiments, the plasma treatment device 100 includes sensors 140 that measure plasma parameters. The plasma parameters may include measurements of the electric current discharged into the biological surface 210, the voltage drop between the dielectric barrier 116 and the surface 210. The plasma parameters may include one or more parameters indicative of the energy density of the plasma 118, such as the spectrum of light emitted by the plasma 118, the ion-density in the plasma 118, or variation in time of the prior-mentioned parameters that would indicate non-uniform surface treatment. Without being bound to theory, it is believed that one or more short-lived discontinuities in the discharge voltage or discharge current indicates a non-uniformity in the form of preference of the cold atmospheric plasma 118 for one or more highly localized ion-rich regions on the surface.

In some embodiments, one or more sensors 140, placed on the surface 210 at or near the treatment region, measure parameters of the plasma 118 or of the biological surface 210. For example, the plasma treatment device 100 may include ion sensors, such as pH sensors or chloride sensors, light sensors, reactive oxygen sensors, a surface temperature sensor, a distance sensor, humidity sensors, etc.

In some embodiments, sensors 140 placed either on the surface 210 or on the plasma treatment device 100 measure the ambient environment. Such sensors 140 may include ion sensors, light sensors, reactive oxygen sensors, temperature sensors, humidity sensors, etc.

In some embodiments, a position reference sensor placed on the plasma treatment device 100 is operably coupled to a distance sensor on the biological surface 210. The position reference sensor may determine the distance of the dielectric barrier 118 from the surface 210. In some embodiments, a distance sensor, such as a laser rangefinder included in the plasma treatment device 100, measures the distance from the dielectric barrier 118 to the surface 210.

In some embodiments, the sensors 140 communicate with the controller 142, as part of the plasma source 110. The controller 140 may be operably coupled to the plasma treatment device 100, and may receive input from the sensors 140 and process that input to determine control data for the plasma treatment device 100. In some embodiments, the control data includes, but is not limited to, signals sent to electronic components of the plasma treatment device 100 to modulate the current or the voltage provided to the electrode 116, and signals sent to other components of the plasma treatment device 100 to produce a perceptible signal. In some embodiments, the perceptible signal is a haptic feedback or an audible or visible indicator. In some embodiments, the controller 142 sends control data in response to an unsafe dose of energy or reactive species produced by the plasma 118.

As previously described, without being bound to theory, a plasma dose is believed to determine exposure to one or more plasma generated species such as, reactive chemical species, energetic species including ions and electrons, photons, etc.

In some embodiments, a plasma dose is a concentration of a given species imparted to a given region on the biological surface 210 over a period of time, expressed as a number per unit-area, per unit-time (such as "per square-centimeter seconds"). In some embodiments, the controller 142 determines a treatment duration and control data to send to the plasma treatment device 100, by integrating the plasma dose over the area of the dielectric barrier 116, to provide a plasma dose per unit time.

In some embodiments, when the plasma treatment device 100 remains over a given region on the biological surface 210 for a length of time such that the plasma 118 is likely to harm the surface 210, the plasma treatment is considered unsafe. Conversely, in some embodiments, if the treatment device 100 remains over the given region for a length of time such that the plasma 118 is unlikely to have the desired effect, the plasma treatment is considered to have provided an ineffective dose. In some embodiments, these doses are not unique values, but rather are thought to occur in ranges. As such, a controller 142 may determine an unsafe range or an ineffective range of doses, wherein it will send control data to the plasma treatment device 100 to produce a perceptible signal or to modulate the plasma 118, or both.

In some embodiments, the controller 142 responds to an unsafe dose by sending a signal for the source to be moved away from the region on the biological surface 210 toward a second region. The controller 142 may respond to an unsafe dose by sending control data to the electronic components of the plasma treatment device 100 to turn off the plasma 118, or to modulate the power provided to the electrode 114 to diminish the generation of energetic species and reactive species in the plasma 118.

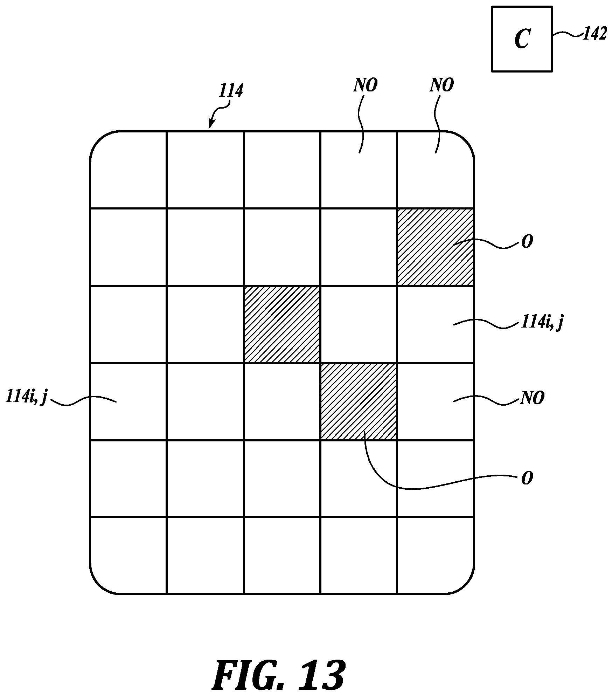

In some embodiments the plasma 118 is generated by a plurality of pixelated electrodes 114i,j arranged in a matrix, as shown in FIG. 13. The pixelated electrodes may be individually addressable by the controller 142, where the controller determines a discharge power for a given pixelated electrode 114. In some embodiments, the controller uses input from current and voltage sensors 140 for the pixelated electrodes 114i,j to counteract non-uniform plasma 118 constriction or localization. In some embodiments, when the plasma 118 localizes to a spot on the biological surface 210 having disparate chemical or physical properties, the controller 142 receives input indicating which pixelated electrodes 114i,j are drawing a disproportionate rate of electrical power, relative to the average for the matrix 114. The controller 142 may modulate the plasma 118 by turning off the electrodes 114i,j that are drawing excess power, thereby distributing plasma energy to operational electrodes O, and diminishing the undesirable effects of plasma non-uniformity near the non-operational electrodes NO.

The components of the cold plasma system 100 may communicate directly through wired and powered connections. These components may communicate to each other via a network (not shown), which may include suitable communication technology including, but not limited to, wired technologies such as DSL, Ethernet, fiber optic, USB, and Firewire; wireless technologies such as WiFi, WiMAX, 3G, 4G, LTE, and Bluetooth; and the Internet.

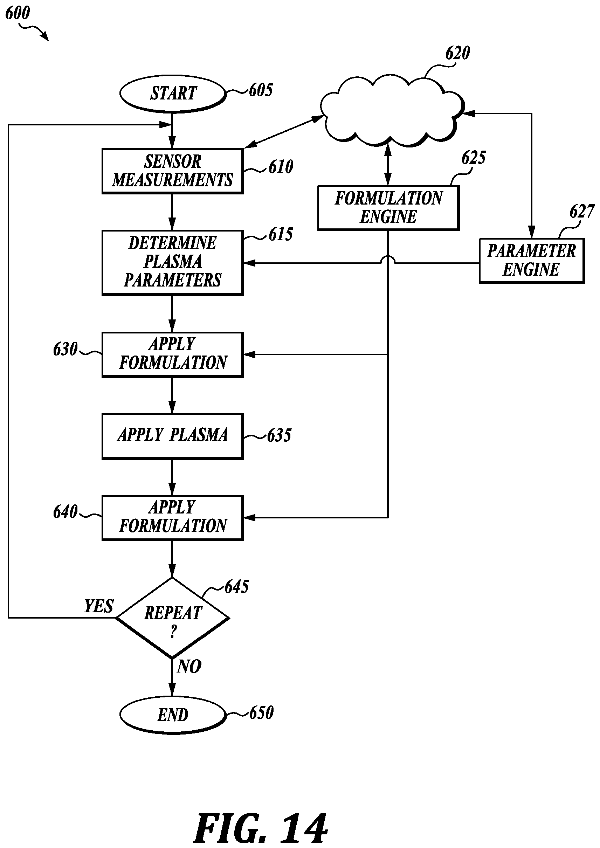

In some embodiments, the controller 142 includes a non-transitory computer readable medium having computer executable instructions and data stored thereon that cause, in response to execution by one or more processors of a computing device, the computing device to implement a method of treatment 600 as described herein and illustrated in Fig. 14.

FIG. 14 is a flowchart of a method of cold plasma treatment not according to the invention but present for illustration purposes only. In some embodiments, the method of treatment 600 of the region of the biological surface 210 with the cold atmospheric plasma 118 includes generating the cold plasma between the plasma treatment device 100 and the region. The method of treatment 600 may include measuring one or more treatment parameters with one or more sensors 140 and determining a plasma dose from the treatment parameters. In some embodiments, the method of treatment 600 includes modulating one or more of the treatment parameters to adjust the plasma dose, and switching off the cold atmospheric plasma 118.

In some embodiments, the method may include additional steps or may be practiced without all steps illustrated in the flow chart. The method starts at block 605, and proceeds to block 610 where one or more sensors 140 measure treatment parameters, for example, ambient parameters and surface parameters. In some embodiments, prior to generating the plasma 118, the method 600 includes placing at least one sensor 140 onto the biological surface 210 at or near the region. As previously described, the sensors 140 may be operably coupled to the controller 142, and may provide sensor input to the controller 142 to be used in block 615 to determine plasma parameters necessary for effective treatment. In some embodiments, the plasma parameters are defined by default values, and the controller 142 does not act until the plasma 118 has been turned on. In some embodiments, the plasma parameters include a discharge voltage as a function of time, a discharge current as a function of time, a plasma temperature as a function of time, or a gas temperature near the region as a function of time. In some embodiments, the sensor measurements are provided to a data storage system 620, which may aggregate the measurements with other sensor data. In some embodiments, a parameter engine communicates parameter information to the controller 142 as shown in block 627. The parameter engine determines a treatment dose based on aggregate sensor inputs accumulated and stored in a data storage system 620, and further determines a set of plasma parameters that are provided to the controller 142.

In block 630, a cosmetic formulation is applied to the treatment region. In some embodiments, the cosmetic formulation enhances plasma treatment. In some embodiments, the cosmetic formulation protects the biological surface 210 from harmful aspects of the plasma 118. A formulation engine, shown in block 625, may determine the formulation, which may receive input from the data storage system 620. In some embodiments, the formulation engine applies machine learning to optimize the components of the formulation for a given purpose such as radical scavenging, UV absorption, electrical conductivity, thermal conductivity, etc.

In block 635, the plasma treatment device 100 applies the cold atmospheric plasma 118 to the biological surface 210 at the treatment region. In block 635, a post-plasma formulation is applied to the treatment region of the biological surface 210. As in block 630, the formulation may be determined by a formulation engine as shown in block 625. In some embodiments, the post-plasma formulation may be the same as the formulation of block 630. In some embodiments, the post-plasma formulation may be different from the formulation of block 630. In some embodiments, the post-plasma formulation neutralizes ions and moisturizes the biological surface 210. In some embodiments, the post-plasma formulation counteracts possible oxidative effects of plasma treatment by including anti-oxidant ingredients.

In block 645, the treatment may be repeated. In some embodiments, the controller 142 determines whether the treatment dose has been met at block 645. Where the treatment dose has not been met, the controller 142 may repeat the sensor measurements, determine new plasma parameters, and modulate the plasma to provide an effective and safe dose of plasma generated species. In some embodiments, the treatment is not repeated, and the method ends in block 650.

The controller 142 may determine plasma parameters from a group including a current provided to the electrode 114, a driving frequency, a voltage waveform, a peak to peak voltage, a root mean square voltage, a plasma temperature, a gas temperature, optical emission from the plasma 118, etc.

In some embodiments, the controller determines an indicator of uniformity of the cold atmospheric plasma 118. As previously described, uniformity describes the spatial distribution of plasma 118 between the second side of the dielectric barrier 116 and the biological surface 210, as well as whether a time-averaged flow of current between the two surfaces is evenly spread across the treated region on the biological surface 210. In some embodiments, the controller sends control data to the plasma treatment device 100 to modulate one or more of the plasma parameters in response to changes in the indicator of uniformity. The controller may determine the indicator of uniformity intermittently, based on sensor inputs provided to the controller 142.

As understood by one of ordinary skill in the art, a "data storage system" as described herein may be any suitable device configured to store data for access by a computing device. An example of the data storage system 620 is a high-speed relational database management system (DBMS) executing on one or more computing devices and being accessible over a high-speed network. However, other suitable storage techniques and/or devices capable of providing the stored data in response to queries may be used, and the computing device may be accessible locally instead of over a network, or may be provided as a cloud-based service. The cloud storage system 620 may also include data stored in an organized manner on a computer-readable storage medium.

In general, the word "engine," as used herein, refers to logic software and algorithms embodied in hardware or software instructions, which can be written in a programming language, such as C, C++, COBOL, JAVA^{™}, PHP, Perl, HTML, CSS, JavaScript, VBScript, ASPX, Microsoft .NET^{™}, PYTHON, and/or the like. An engine may be compiled into executable programs or written in interpreted programming languages. Software engines may be callable from other engines or from themselves. Generally, the engines described herein refer to logical modules that can be merged with other engines, or can be divided into sub engines. The engines can be stored in any type of computer readable medium or computer storage device and be stored on and executed by one or more general purpose computers, thus creating a special purpose computer configured to provide the engine or the functionality thereof.

Many embodiments of the technology described above may take the form of computer- or controller-executable instructions, including routines executed by a programmable computer or controller. Those skilled in the relevant art will appreciate that the technology can be practiced on computer/controller systems other than those shown and described above. The technology can be embodied in a special-purpose computer, application specific integrated circuit (ASIC), controller or data processor that is specifically programmed, configured or constructed to perform one or more of the computer-executable instructions described above. Of course, any logic or algorithm described herein can be implemented in software or hardware, or a combination of software and hardware.

From the foregoing, it will be appreciated that specific embodiments of the technology have been described herein for purposes of illustration, but that various modifications may be made without deviating from the disclosure. Moreover, while various advantages and features associated with certain embodiments have been described above in the context of those embodiments, other embodiments may also exhibit such advantages and/or features, and not all embodiments need necessarily exhibit such advantages and/or features to fall within the scope of the invention. Additionally, steps may be performed in any suitable order. Accordingly, the disclosure can encompass other embodiments not expressly shown or described herein.

For the purposes of the present disclosure, lists of two or more elements of the form, for example, "at least one of A, B, and C," is intended to mean (A), (B), (C), (A and B), (A and C), (B and C), or (A, B, and C), and further includes all similar permutations when any other quantity of elements is listed.

## Claims

1. A cold plasma system (100) for cosmetic treatment of a region of a biological surface (210), comprising:
a cold plasma generator comprising:
an electrode (114, 114i,j, 116, 142), and
a dielectric barrier (16, 116) having a first side that faces the electrode (114, 114i,j, 116, 142) and a second side that faces away from the electrode (114, 114i,j, 116, 142); and
an auxiliary treatment device configured to enhance effects of the cold plasma on the region, **characterized in that** the auxiliary treatment device includes actuating members (120) configured to move in opposite directions to each other and parallel to the biological surface for compressing or stretching the biological surface (210) during application of the cold plasma.

2. The system of claim 1, wherein the auxiliary treatment device is selected from a group consisting of (i) a vibration device (130), (ii) a source of ultrasound, (iii) a light source (150) configured to illuminate the region, (iv) a source of air directed to the region; and (v) a source of heat directed to the region.

3. The system of claim 1, wherein the cold plasma generator comprises:
a plasma generator body; and
a head (31, 110, 110a-110d, 110-x) that is removably attached to the plasma generator body, wherein the head (31, 110, 110a-110d, 110-x) has a mounting side facing the plasma generator body and an application side that carries the electrode (114, 114i,j, 116, 142) and the dielectric barrier (16, 116).

4. The system of claim 1, wherein the electrode (114, 114i,j, 116, 142) is pixelated into individually activated areas capable of generating the cold plasma, the system further comprising:
a controller (320, 142) configured to energize the activated areas of the electrode (114, 114i,j, 116, 142).

5. The system of claim 3, wherein the head (31, 110, 110a-110d, 110-x) tapers from a larger size at the mounting side of the head (31, 110, 110a-110d, 110-x) to a smaller size at the application side of the head (31, 110, 110a-110d, 110-x).

6. The system of claim 3, wherein the head (31, 110, 110a-110d, 110-x) tapers from a larger size at the application side of the head (31, 110, 110a-110d, 110-x) to a smaller size at the mounting side of the head (31, 110, 111a-110d, 110-x).

7. The system of claim 3, wherein the head (31, 110, 110a-110d, 110-x) has a formula reservoir (180) configured to provide a formula to the biological surface (210) via one or more conduits (184).

8. The system of claim 7, wherein the formula reservoir (180) is removable and interchangeable, and wherein the formula reservoir (180) contains a prepared formula.

9. The system of claim 7, wherein the prepared formula is selected from a group consisting of a fragrance, a pigment, a cream, an oil, a natural extract, a moisturizer, astringents, pharmaceutically active compounds, acid neutralizing creams, anti-oxidants, a clarifying cream, and an acidity buffering cream.

10. The system of claim 3, wherein the head (31, 110, 110a-110d, 110-x) includes a flexible skirt (170) at the application side of the head (31, 110, 110a-110d, 110-x).

11. The system of claim 3, wherein the head (31, 110, 110a-110d, 110-x) includes a conformable material at the application side of the head (31, 110, 110a-110d, 110-x), wherein the conformable material is configured to reversibly conform to contours of the region of the biological surface (210) that faces the application side of the head (31, 110, 110a-110d, 110-x).

## Patentansprüche

1. Kaltplasmasystem (100) zur kosmetischen Behandlung eines Bereichs einer biologischen Oberfläche (210), Folgendes umfassend:
einen Kaltplasmagenerator, der Folgendes umfasst:
eine Elektrode (114, 114i,j, 116, 142) und
eine dielektrische Barriere (16, 116) mit einer ersten Seite, die der Elektrode (114, 114i,j, 116, 142) zugewandt ist, und einer zweiten Seite, die der Elektrode (114, 114i,j, 116, 142) abgewandt ist; und
eine Behandlungshilfsvorrichtung, die dazu ausgestaltet ist, Wirkungen des Kaltplasmas auf den Bereich zu verstärken, **dadurch gekennzeichnet, dass** die Behandlungshilfsvorrichtung Betätigungselemente (120) umfasst, die dazu ausgestaltet sind, sich in einander entgegengesetzte Richtungen und parallel zu der biologischen Oberfläche zu bewegen, um die biologische Oberfläche (210) während der Anwendung des Kaltplasmas zusammenzudrücken oder zu dehnen.

2. System nach Anspruch 1, wobei die Behandlungshilfsvorrichtung ausgewählt ist aus einer Gruppe bestehend aus (i) einer Vibrationsvorrichtung (130), (ii) einer Ultraschallquelle, (iii) einer Lichtquelle (150), die zum Beleuchten des Bereichs ausgestaltet ist, (iv) einer Quelle von Luft, die auf den Bereich gerichtet ist; und (v) einer Quelle von wärme, die auf den Bereich gerichtet ist.

3. System nach Anspruch 1, wobei der Kaltplasmagenerator Folgendes umfasst:
einen Plasmageneratorkörper; und
einen Kopf (31, 110, 110a - 110d, 110-x), der entfernbar an dem Plasmageneratorkörper angebracht ist, wobei der Kopf (31, 110, 110a - 110d, 110-x) eine Befestigungsseite, die dem Plasmageneratorkörper zugewandt ist, und eine Anwendungsseite, welche die Elektrode (114, 114i,j, 116, 142) und die dielektrische Barriere (16, 116) trägt, aufweist.

4. System nach Anspruch 1, wobei die Elektrode (114, 114i,j, 116, 142) in einzelne aktivierte Teilbereiche pixeliert ist, die zum Erzeugen des Kaltplasmas in der Lage sind, wobei das System ferner Folgendes umfasst: eine Steuerung (320, 142), die dazu ausgestaltet ist, an die aktivierten Teilbereiche der Elektrode (114, 114i,j, 116, 142) eine Spannung anzulegen.

5. System nach Anspruch 3, wobei der Kopf (31, 110, 110a - 110d, 110-x) sich von einer größeren Größe auf der Befestigungsseite des Kopfes (31, 110, 110a - 110d, 110-x) zu einer kleineren Größe auf der Anwendungsseite des Kopfes (31, 110, 110a - 110d, 110-x) verjüngt.

6. System nach Anspruch 3, wobei der Kopf (31, 110, 110a - 110d, 110-x) sich von einer größeren Größe auf der Anwendungsseite des Kopfes (31, 110, 110a - 110d, 110-x) zu einer kleineren Größe auf der Befestigungsseite des Kopfes (31, 110, 110a - 110d, 110-x) verjüngt.

7. System nach Anspruch 3, wobei der Kopf (31, 110, 110a - 110d, 110-x) ein Formulierungsreservoir (180) aufweist, das dazu ausgestaltet ist, der biologischen Oberfläche (210) über eine oder mehrere Leitungen (184) eine Formulierung bereitzustellen.

8. System nach Anspruch 7, wobei das Formulierungsreservoir (180) entfernbar und auswechselbar ist und wobei das Formulierungsreservoir (180) eine zubereitete Formulierung enthält.

9. System nach Anspruch 7, wobei die zubereitete Formulierung ausgewählt ist aus einer Gruppe bestehend aus einem Duftstoff, einem Pigment, einer Creme, einem Öl, einem natürlichen Extrakt, einem Feuchtigkeitsspender, Adstringenzien, pharmazeutisch wirksamen Verbindungen, Säure neutralisierenden Cremes, Antioxidanzien, einer Creme gegen Unreinheiten und einer Säure puffernden Creme.

10. System nach Anspruch 3, wobei der Kopf (31, 110, 110a - 110d, 110-x) auf der Anwendungsseite des Kopfes (31, 110, 110a - 110d, 110-x) eine flexible Umrandung (170) umfasst.

11. System nach Anspruch 3, wobei der Kopf (31, 110, 110a - 110d, 110-x) auf der Anwendungsseite des Kopfes (31, 110, 110a - 110d, 110-x) ein anpassbares Material umfasst, wobei das anpassbare Material dazu ausgestaltet ist, sich reversibel an Konturen des Bereichs der biologischen Oberfläche (210), die der Anwendungsseite des Kopfes (31, 110, 110a - 110d, 110-x) zugewandt ist, anzupassen.

## Revendications

1. Système à plasma froid (100) pour traitement cosmétique d'une région d'une surface biologique (210), comprenant :
un générateur de plasma froid comprenant :
une électrode (114, 114i,j, 116, 142), et
une barrière diélectrique (16, 116) présentant un premier côté qui fait face à l'électrode (114, 114i,j, 116, 142) et un deuxième côté qui est tourné à l'opposé de l'électrode (114, 114i,j, 116, 142) ; et
un dispositif de traitement auxiliaire configuré pour améliorer les effets du plasma froid sur la région, **caractérisé en ce que** le dispositif de traitement auxiliaire comporte des éléments d'actionnement (120) configurés pour se déplacer dans des directions opposées l'une à l'autre et parallèlement à la surface biologique pour comprimer ou étirer la surface biologique (210) pendant l'application du plasma froid.

2. Système selon la revendication 1, dans lequel le dispositif de traitement auxiliaire est sélectionné à partir d'un groupe consistant en (i) un dispositif à vibration (130), (ii) une source d'ultrasons, (iii) une source de lumière (150) configurée pour éclairer la région, (iv) une source d'air dirigé vers la région ; et (v) une source de chaleur dirigée vers la région.

3. Système selon la revendication 1, dans lequel le générateur de plasma froid comprend :
un corps de générateur de plasma ; et
une tête (31, 110, 110a-110d, 110-x) qui est attachée de manière amovible au corps de générateur de plasma, dans lequel la tête (31, 110, 110a-110d, 110-x) présente un côté montage faisant face au corps de générateur de plasma et un côté application qui porte l'électrode (114, 114i,j, 116, 142) et la barrière diélectrique (16, 116).

4. Système selon la revendication 1, dans lequel l'électrode (114, 114i,j, 116, 142) est pixelisée en zones individuellement activées aptes à générer le plasma froid, le système comprenant en outre :
un dispositif de commande (320, 142) configuré pour exciter les zones activées de l'électrode (114, 1141,j, 116, 142).

5. Système selon la revendication 3, dans lequel la tête (31, 110, 110a-110d, 110-x) se resserre depuis une taille plus grande au niveau du côté montage de la tête (31, 110, 110a-110d, 110-x) vers une taille plus petite au niveau du côté application de la tête (31, 110, 110a-110d, 110-x).

6. Système selon la revendication 3, dans lequel la tête (31, 110, 110a-110d, 110-x) se resserre depuis une taille plus grande au niveau du côté application de la tête (31, 110, 110a-110d, 110-x) vers une taille plus petite au niveau du côté montage de la tête (31, 110, 110a-110d, 110-x).

7. Système selon la revendication 3, dans lequel la tête (31, 110, 110a-110d, 110-x) possède un réservoir de formule (180) configuré pour fournir une formule à la surface biologique (210) via un ou plusieurs conduits (184).

8. Système selon la revendication 7, dans lequel le réservoir de formule (180) est amovible et interchangeable, et dans lequel le réservoir de formule (180) contient une formule préparée.

9. Système selon la revendication 7, dans lequel la formule préparée est sélectionnée à partir d'un groupe consistant en un parfum, un pigment, une crème, une huile, un extrait naturel, un hydratant, des astringents, des composés pharmaceutiquement actifs, des crèmes de neutralisation d'acide, des antioxydants, une crème clarifiante et une crème tampon d'acidité.

10. Système selon la revendication 3, dans lequel la tête (31, 110, 110a-110d, 110-x) comporte une collerette souple (170) au niveau du côté application de la tête (31, 110, 110a-110d, 110-x).

11. Système selon la revendication 3, dans lequel la tête (31, 110, 110a-110d, 110-x) comporte un matériau conformable au niveau du côté application de la tête (31, 110, 110a-110d, 110-x), dans lequel le matériau conformable est configuré pour se conformer de manière réversible aux contours de la région de la surface biologique (210) qui fait face au côté application de la tête (31, 110, 110a-110d, 110-x).
